Europäisches Patentamt

European Patent Office

Office européen des brevets

(11) Veröffentlichungsnummer: **0 308 690**
**A1**

## (12) EUROPÄISCHE PATENTANMELDUNG

(21) Anmeldenummer: **88114078.4**

(22) Anmeldetag: **29.08.88**

(51) Int. Cl.4: **A61B  17/39**

The title of the invention has been amended (Guidelines for Examination in the EPO, A-III, 7.3).

(30) Priorität: **11.09.87 DE 3730603**

(43) Veröffentlichungstag der Anmeldung:
**29.03.89 Patentblatt 89/13**

(84) Benannte Vertragsstaaten:
**AT BE DE FR GB IT**

(71) Anmelder: **Siemens Aktiengesellschaft Berlin und München**
**Wittelsbacherplatz 2**
**D-8000 München 2(DE)**

(72) Erfinder: **Redler, Udo, Dipl.-Ing.**
**Hans-Sachs-Strasse 20**
**D-8521 Effeltrich(DE)**
Erfinder: **Hagen, Uwe, Dipl.-Ing. (FH)**
**Auf der Hut 25**
**D-8550 Forchheim(DE)**

(54) **Aus mindestens drei Teilelektroden bestehende neutrale Elektrode für ein HF-Chirurgiegerät.**

(57)  Die Erfindung betrifft eine mindestens dreiteilige Elektrode, insbesondere eine neutrale Elektrode (10) für ein HF-Chirurgiegerät (2). Die Teilelektroden (12a, 14a, 16a; 12b, 14b, 16b) sind auf einem gemeinsamen biegsamen Träger (11) angeordnet; sie sind dabei symmetrisch und drehversetzt bezüglich einer senkrecht auf dem Träger (11) stehenden Symmetrieachse (A) angeordnet. Dadurch wird erreicht, daß die Applikation der Elektrode (10) bezüglich einer Drehung um die Achse (A) am Patienten (P) unkritisch ist. Es ist damit eine sichere Überwachung auf korrektes Anliegen am Patienten (P) gewährleistet.

FIG 2

EP 0 308 690 A1

## Mindestens dreiteilige Elektrode für ein HF-Chirurgiegerät

Die Erfindung bezieht sich auf eine mindestens dreiteilige Elektrode, insbesondere neutrale Elektrode für ein HF-Chirurgiegerät, bei der die Teilelektroden auf einem gemeinsamen biegsamen Träger angeordnet sind.

Eine neutrale Elektrode der eingangs genannten Art, die mit drei flächenhaften Teilelektroden arbeitet, ist beispielsweise aus der DE-OS 35 44 443 ( = VPA 85 P 8546 DE) bekannt.

Es hat sich im Zusammenhang mit HF-Chirurgiegeräten erwiesen, daß eine mehrstückige Ausführung einer neutralen Elektrode wünschenswert ist, wenn mittels einer Überwachungsschaltung nachgewiesen werden soll, ob die neutrale Elektrode mit großer Fläche und nicht nur etwa punktförmig am Patienten bei der HF-chirurgischen Behandlung anliegt. Eine solche Überwachungsschaltung für eine dreistückige neutrale Elektrode wird in FIG 4 der bereits erwähnten DE-OS 35 44 443 gezeigt und im Text näher beschrieben.

Wird als mehrteilige Flachelektrode eine solche beispielsweise mit rechteckförmigen Teilelektroden verwendet und wird diese Flachelektrode am Oberschenkel des Patienten appliziert, so sind dabei im Prinzip zwei Applikationsrichtungen möglich. In der einen Applikationsrichtung sind die drei Teilelektroden in Richtung der Längsachse des Oberschenkels ausgerichtet. In diesem Fall wird man von einer falschen Applikation sprechen müssen, denn die vom Operationsfeld, z.B. im abdominalen Bereich, ausgehende Hochfrequenz wird die drei Teilelektroden in unter schiedlicher Intensität erreichen. Dieses kann bezüglich der Überwachung einer korrekten Anlage der neutralen Elektrode am Patienten zu einer Fehlmessung führen. Im anderen Fall sind die drei Teilelektroden quer zur Längsrichtung des Oberschenkels appliziert. In diesem Fall wird man von einer richtigen Applikation sprechen können, denn in diesem Fall werden die drei Teilelektroden von der vom Operationsfeld ausgehenden Hochfrequenz etwa mit gleicher Intensität erreicht, wobei gleiche Flächengröße vorausgesetzt ist.

Im Krankenhaus wird die Applikation von neutralen Elektroden häufig von Hilfspersonal durchgeführt. Um hier eine Fehlermöglichkeit und damit einen Zeitverlust bei der Vorbereitung des Patienten auf die Operation zu vermeiden, sollte nach einer Möglichkeit gesucht werden, wie die einzelnen Teilelektroden -unabhängig von der Orientierung der neutralen Elektrode zur Längsachse beispielsweise des Oberschenkels - mit gleicher HF-Leistung beaufschlagt werden. In erster Näherung soll dabei davon ausgegangen werden, daß alle Teilelektroden dieselbe Fläche besitzen.

Der Erfindung liegt somit die Aufgabe zugrunde, eine mindestens dreiteilige Elektrode der eingangs genannten Art so auszugestalten, daß eine weitgehend gleichmäßige Verteilung der vom Operationsfeld ausgehenden HF-Energie auf die Teilelektroden erzielt wird. Dabei soll es weitgehend gleichgültig sein, in welcher Orientierung bezüglich ihrer Normale die Elektrode am Patienten appliziert wird.

Diese Aufgabe wird erfindungsgemäß dadurch gelöst, daß die Teilelektroden symmetrisch zu einer senkrecht auf dem Träger stehenden Symmetrieachse und bezüglich der Symmetrieachse in Umfangsrichtung gegeneinander versetzt angeordnet sind.

Um eine leichte Applikation zu erreichen, sollten die Teilelektroden zumindest teilweise gekrümmte Kanten aufweisen. Die Teilelektroden können dabei insbesondere rund, aber auch sektorförmig ausgebildet sein.

Im allgemeinen Fall wird man so vorgehen, daß die Teilelektroden flächenmäßig gleich groß sind. In Sonderfällen kann es jedoch auch angebracht sein, die eine oder andere Teilelektrode unterschiedlich groß auszubilden. Dies hängt von der geometrischen Konfiguration ab.

Weitere vorteilhafte Ausgestaltungen sind in den Unteransprüchen gekennzeichnet.

Ausführungsbeispiele der Erfindung werden im folgenden anhand von drei Figuren näher erläutert. Es zeigen:

FIG 1 die falsche Applikation einer neutralen Elektrode an einem Patienten gemäß dem Stande der Technik;

FIG 2 in Aufsicht eine erste Ausführungsform einer erfindungsgemäßen Elektrode mit drei symmetrisch verteilten kreisrunden Teilelektroden; und

FIG 3 in Aufsicht eine zweite erfindungsgemäße Ausführungsform einer neutralen Elektrode mit drei sektorförmigen Teilelektroden.

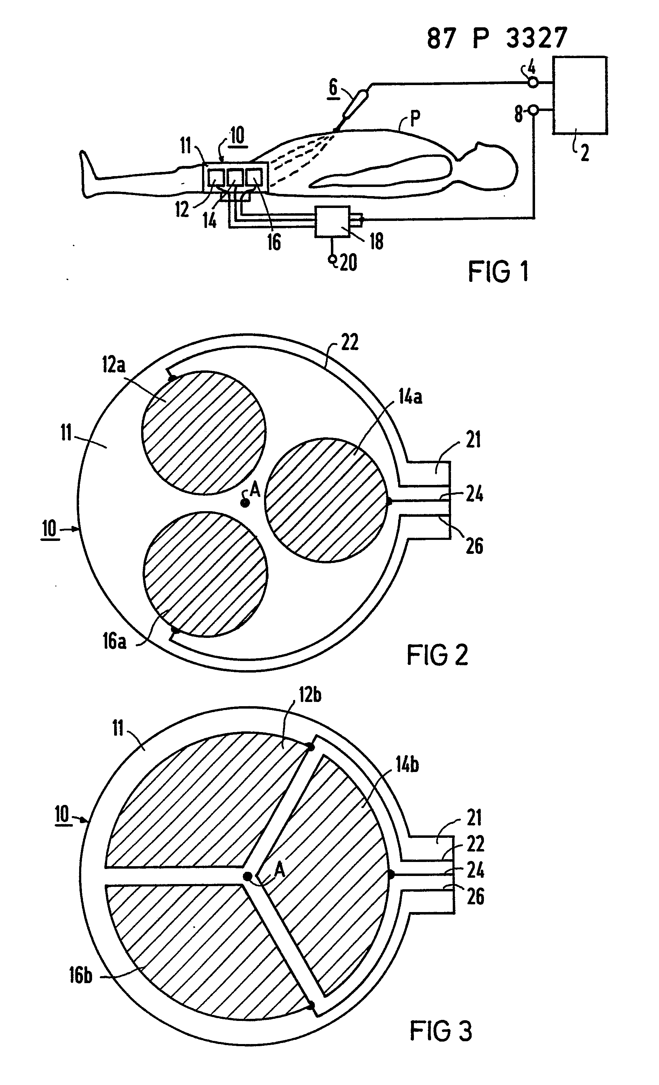

Nach FIG 1 umfaßt ein HF-Chirurgiegerät 2 einen Anschluß 4 für die aktive Elektrode 6 und einen weiteren Anschluß 8 für die neutrale Elektrode 10, die vorliegend am Oberschenkel eines Patienten P befestigt ist. Die neutrale Elektrode 10 ist im vorliegenden Fall in drei Teilelektroden 12, 14 und 16 unterteilt. Der Chirurg führt beim Eingriff die aktive Elektrode 6 und nimmt gezielt Koagulationen oder Schnitte vor.

Dem HF-Chirurgiegerät 2 ist eine Schaltungsanordnung 18 zur Überwachung der neutralen Elektrode 10 zugeordnet. Diese Schaltungsanordnung 18 ist eine Sicherheitsschaltung, die es festzustellen erlaubt, ob die neutrale Elektrode 10 mit ausreichend großer Fläche am Patienten P anliegt. Ist dieses nicht der Fall, so gibt sie an einem Ausgang 20 ein Warnsignal ab. Die Sicherheitsschaltung 18 kann gemäß der DE-OS 35 44 443 (= VPA 85 P 8546 DE) aufgebaut sein.

In FIG 1 ist angenommen, daß die Teilelektroden 12, 14, 16 von rechteckigem Querschnitt sind und auf einem Träger 11 in Längsrichtung des Oberschenkels angeordnet sind. In diesem Fall kann man bei Einschalten des HF-Chirurgiegerätes 2 keine gleichmäßige Verteilung der HF-Energie auf die drei Teilelektroden 12, 14, 16 erwarten. Infolge dieser unsymmetrischen Durchströmung der Teilelektroden 12, 14, 16 mit HF-Energie ist unter Umständen keine sichere Überwachung der korrekten Anlage der neutralen Elektrode 10 möglich.

Um dieser Schwierigkeit abzuhelfen, ist nach den Figuren 2 und 3 jeweils eine dreiteilige Elektrode 10 konzipiert, bei der die drei Teilelektroden 12a, 14a, 16a bzw. 12b, 14b, 16b zu einer senkrecht auf dem Träger 11 stehenden Rotationssymmetrieachse A symmetrisch und gegeneinander drehversetzt angeordnet sind.

Nach FIG 2 sind die drei gleichen Teilelektroden 12a, 14a, 16a, die auf dem Träger 11 aus einem biegsamen, elektrisch isolierenden Material befestigt sind, im wesentlichen kreisrund ausgebildet. Sie bestehen jeweils aus einer Metallfolie oder aus einem Metallnetz. Der Träger 11, der bevorzugt selbstklebend ausgeführt ist und/oder aus einem Gummi bestehen kann, ist ebenfalls im wesentlichen rund ausgebildet. Die äußeren Verbindungslinien der drei Teilelektroden 12a, 14a, 16a bilden eine im wesentlichen dreieckige Auflagefläche für den Patienten P. Die Teilelektroden 12a, 14a, 16a sind also jeweils um 120° bezüglich der Symmetrieachse A in Umfangsrichtung gegeneinander versetzt angeordnet. Die Achse A steht zentral und senkrecht auf dem Träger 11.

Am rechten Teil des runden Trägers 11 ist eine kleine Stütze oder Lasche 21 für einen elektrischen Leitungsanschluß vorgesehen. Dieser Leitungsanschluß nimmt drei Verbindungsleitungen 22, 24, 26 für die Teilelektroden 12, 14 bzw. 16 auf. Wie ersichtlich, sind die drei elektrischen Leitungen 22, 24 und 26 in der Lasche 21 eng benachbart und parallel zueinander geführt. Dadurch ist es möglich, an dieser Stelle eine Klammer angreifen zu lassen, die die Verbindung der drei Leitungen zur Sicherheitsschaltung 18 herstellt. Außerdem ist bemerkenswert, daß die beiden äußeren Verbindungsleitungen 22, 26 gleich lang sind, was die Herstellung vereinfacht und die Vorratskosten gering hält. Infolge des gleich langen und symmetrischen Aufbaus ergeben sich gleiche parasitäre Kapazitäten, und daraus resultiert eine weitgehende Unabhängigkeit von Fertigungstoleranzen.

Es soll noch hervorgehoben werden, daß die drei Teilelektroden 12a, 14a und 16a in FIG 2 flächenmäßig gleich groß ausgebildet sind.

Wird die in FIG 2 dargestellte neutrale Elektrode 10 am Oberschenkel des Patienten P (vergleiche FIG 1) appliziert, so ist es weitgehend gleichgültig, in welcher Positionierung bezüglich einer Drehung um die Symmetrieachse A dies geschieht. In allen Positionierungen wird eine weitgehend gleichmäßige Verteilung der HF-Energie auf die Teilelektroden 12a, 14a, 16a erzielt. Der Unterschied ist maximal durch einen Winkel von 60° gegeben. Sind mehr Teilelektroden vorhanden, beispielsweise statt drei insgesamt vier, so wird der Unterschiedswinkel sogar noch kleiner, beispielsweise also 45°.

Bei der Ausführungsform einer dreiteiligen Elektrode 10 nach FIG 3 sind insgesamt drei sektorförmig ausgebildete Teilelektroden 12b, 14b und 16b auf dem elektrisch isolierenden Träger 11 angeordnet. Die drei Teilelektroden 12b, 14b, 16b bestehen vorliegend aus drei gleichflächigen Kreissegmenten, die jeweils einen Winkel von 120° umfassen. Jede Teilelektrode hat somit eine runde und zwei gerade Kanten. Auch hier ist eine rotationssymmetrische Anordnung bezüglich der senkrecht auf dem Träger 11 stehenden Symmetrieachse A getroffen. Der Winkel der Drehversetzung beträgt auch hier wieder 120°. Auch diese Elektrode 10 ist bevorzugt selbstklebend ausgebildet.

Bezüglich der Applikation am Patienten P ergeben sich hier dieselben Vorteile wie bei der Elektrode 10 nach FIG 2.

Die Rotationssymmetrie bei den Ausführungsformen nach FIG 2 und 3 wurde dadurch erreicht, daß die Teilelektroden zumindest teilweise gleichmäßig gekrümmte Umfangslinien aufweisen. Weitere geometrische Formen einer mehrteiligen Elektrode 10 mit gleich großen oder physiologisch bedingt unterschiedlich großen Teilflächen 12, 14, 16 sind denkbar. Die Elektrode 10 könnte auch vier-, fünf-, sechs- usw. -teilig ausgeführt sein, so daß der Winkel der Drehversetzung der einzelnen Teilelektroden dann 90°, 72° bzw. 60° usw. betragen würde.

**Ansprüche**

1. Mindestens dreiteilige Elektrode, insbesondere neutrale Elektrode für ein HF-Chirurgiegerät, bei der die Teilelektroden auf einem gemeinsamen biegsamen Träger angeordnet sind, **dadurch gekennzeichnet,** daß die Teilelektroden (12a, 14a, 16a; 12b, 14b, 16b) symmetrisch zu einer senkrecht auf dem Träger (11) stehenden Symmetrieachse (A) und bezüglich der Symmetrieachse (A) in Umfangsrichtung gegeneinander versetzt angeordnet sind.

2. Elektrode nach Anspruch 1, **dadurch gekennzeichnet,** daß die Teilelektroden (12a, 14a, 16a; 12b, 14b, 16b) zumindest teilweise gleichmäßig gekrümmte Umfangslinien aufweisen.

3. Elektrode nach Anspruch 1 oder 2, **dadurch gekennzeichnet,** daß die Teilelektroden (12a, 14a, 16a) rund ausgebildet sind.

4. Elektrode nach Anspruch 1 oder 2, **dadurch gekennzeichnet,** daß die Teilelektroden (12b, 14b, 16b) sektorförmig ausgebildet sind.

5. Elektrode nach einem der Ansprüche 1 bis 4, **dadurch gekennzeichnet,** daß die Teilelektroden (12a, 14a, 16a; 12b, 14b, 16b) flächenmäßig gleich groß sind.

6. Elektrode nach einem der Ansprüche 1 bis 5, **dadurch gekennzeichnet,** daß der Träger (11) rund ausgebildet ist.

7. Elektrode nach einem der Ansprüche 1 bis 6, **dadurch gekennzeichnet,** daß der Träger (11) mit einer Lasche (21) versehen ist, auf dem an die Teilelektroden (12, 14, 16) angeschlossene Leitungen (22, 24, 26) parallel zueinander verlaufen.

8. Elektrode nach einem der Ansprüche 1 bis 7, **dadurch gekennzeichnet,** daß drei Teilelektroden (12, 14, 16) vorgesehen sind.

9. Elektrode nach einem der Ansprüche 1 bis 8, **dadurch gekennzeichnet,** daß die Symmetrieachse (A) zentral auf dem Träger (11) angeordnet ist.

87 P 3327

FIG 1

FIG 2

FIG 3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | CA-A-1 219 642 (M. FRIZE et al.) <br> * Figuren 2-4; Seite 5, Zeilen 16-25; Seite 6, Zeilen 17-22 * <br> --- | 1-3,6-9 | A 61 B 17/39 |
| A | MEDICAL & BIOLOGICAL ENGINEERING & COMPUTING, Band 24, Nr. 3, Mai 1986, Seiten 311-316, IFMBE, London, GB; M. AUBRY-FRIZE et al.: "Modelling of thermal patterns of electrosurgical dispersive electrodes" <br> * Figur 1; Seite 312, linke Spalte, Absatz 1: "Test electrode" * <br> ----- | 1-3,5,9 | |

**RECHERCHIERTE SACHGEBIETE (Int. Cl.4)**

A 61 B

Der vorliegende Recherchenbericht wurde für alle Patentansprüche erstellt

| Recherchenort | Abschlußdatum der Recherche | Prüfer |
|---|---|---|
| DEN HAAG | 01-12-1988 | VEREECKE A. |